# EUROPEAN PATENT APPLICATION

(11) **EP 4 328 899 A1**
(43) Date of publication of application: **28.02.2024**
(21) Application number: 23186748.2
(22) Date of filing: 20.07.2023
(51) Int. Cl.: G10H 1/00, G10H 1/46, G10H 1/40

(54) **METHOD FOR ADJUSTING AN AUDIO TRACK**

(30) Priority: 02.08.2022 IT 202200016428
(71) Applicant: Pinto, Gianluca, 58031 Arcidosso (IT)
(72) Inventor: Pinto, Gianluca, 58031 Arcidosso (IT)
(74) Representative: Emmi, Mario

(57) **Abstract**

The present invention concerns a method for adjusting an at least one audio track and which is executed by a control unit and a memory unit associated with the control unit, wherein said adjustment method comprises the steps of:
generating at least a first audio track and a second audio track, wherein said first and second audio track are diversified from each other as a function of control parameters selected from:
audio intensity of said first audio track with respect to said second audio track;
time duration of said first audio track with respect to a second audio track;
time interval between said first and second audio track;
or a combination thereof.

## Description

### Scope of the invention

The present invention falls within the scope of audio track control and reproduction devices, in order to execute sound rehabilitation exercises for patients with cognitive and/or memory problems.

### State of the art

Program means used for music therapy are known. In most cases, the latter exploits the effects of music, but does not focus on the linguistic mechanism.

A need is felt to provide a method for adjusting an audio track that allows to execute preparatory auditory exercises for patients with cognitive and/or memory problems, using the so-called negative *mismatch.*

The negative mismatch, also called discordance negativity, is a component of the wave aroused by any event-related potential as a consequence of a discordant stimulus in a sequence.

The variation that produces the negative mismatch, therefore, is a variation that operates a caesura with respect to a 'consolidated' trend, at the level of operational memory.

Given that the mismatch occurs as a direct effect of memory (which would not exist if the one perceived immediately before did not create conflict), both the relationship between the negative mismatch and the time-memory relationship and the potentials that this effect can have, once it has been understood whether and how it can be used, at the level of cognitive rehabilitation are already empirically evident.

The use of the negative mismatch in a rehabilitation function for cognitive purposes is a 2019 discovery by G. Pinto, scientifically proven.

### Summary of the invention

An object of the present invention is to provide a method for adjusting an audio track that allows multiple audio tracks distinct from each other to be reproduced and adjusted according to functional parameters such as duration, intensity and frequency.

These and other purposes are achieved by means of a method for adjusting an audio track and which is executed by a control unit and a memory unit associated with the control unit, wherein said adjustment method comprises the steps of:

generating at least a first audio track and a second audio track, wherein said first and second audio track are diversified from each other as a function of control parameters selected from:
- audio intensity of said first audio track with respect to said second audio track;
- time duration of said first audio track with respect to a second audio track;
- time interval between said first and second audio track;
- or a combination thereof.

In particular, the control parameters are selected from:
- Intensity,
- Quality (tone: waveform),
- Frequency,
- Duration,
- Chronological distance,
- Density,
- or a combination thereof.

The method according to the present invention, therefore, is based on the exclusively perceptual parameters of the sounds:
- Intensity,
- Quality (tone: waveform),
- Frequency,
- Duration
- Chronological distance
- Density.

In this way, the first four are relative to the single sound, the last two are relative to the relationships among more sounds. The Chronological distance is duration of the 'pause' if there is one between sounds. The 'density' is the amount of different sounds heard at the same time. The density is included among the perceptual parameters because the alternation of different densities can create a 'rhythm' or 'arrhythmia' sense, at the perceptual level. The combination of these parameters generates the perception of the musical 'sense' of a sequence of more sounds. The musical language develops, in fact, both vertically (simultaneous sounds of more sounds) and horizontally (sequence of sounds). Harmony and melody are generated from the ensemble of these two dimensions.

The principle, therefore, is to produce a mismatch by varying one or more of these parameters in audio tracks in different and combined ways so as to have a high number of exercises and of difficulty levels that permit the patient's progress.

In this regard, it is necessary to clarify the following:

the single variation that produces the mismatch must b e created artificially. In the case of the intensity or of the 'volume', for example, when it is increased the volume in a musical instrument by playing it physically, the 'tone' is also changed (this is due to the change in the physical pressure, for example, of the bow on the string of a violin, or in the intensity of the percussion of the hammer on the strings of the piano). In order not to have two simultaneous 'modulations' when not required, which would affect the exercise itself, the variation must be constructed artificially by modifying only the *db* on a single audio sample.

In this case the effect of music (i.e. the evocative potential of the millennial elaboration of the language of sounds) has no relevance, actually, in many cases, given the difficulty of concentration of the patients, it is important to be careful to limit its effects: the emblematic examples, just to understand the meaning of these words, is given empirically in the study work carried out with patients that lasted for a few years. One case in particular (similar to all the others) was a patient who was unable to do some exercises, while with others he was. The common feature of the exercises he was unable to carry out all had in common the presence of 'brass' tones. The person in question used to work, before the traumatic event, as an officer in the army, so whenever he listened to themes with an invasive presence of brass the memories that these tones evoked (the musical bands of the armed forces mainly consists of brass and wood) did not allow him to focus on the 'errors' present in the themes, so for the person any sequence was 'right' or 'linear'. This only happened with brass. Once the brass exercises were eliminated from the patient's profile, everything was resolved.

In particular, said first and second track are reproduced in sequence with each other and diversified by varying said control parameters.

Advantageously, program means allow to generate a plurality of audio tracks distinct from each other. In this way, the plurality of distinct audio tracks allows to generate different sound exercises, each with a diversified degree of difficulty.

In particular, each of said control parameters is adjustable by a user via a display screen associated with said control unit.

In this way, the adjustment method makes it possible to obtain diversified audio tracks, in particular by generating a so-called "*Mismatch*", i.e. single or multiple changes in both the material and temporal structure of an audio track. This allows "passive" mental gymnastics and memory training, concerning the time/memory ratio in perception and the perceptual meaning through the sequences of organized sounds, favouring the rehabilitation of the cognitive functions.

Advantageously, on said display screen the audio tracks are represented with respective audio icons and the control parameters with respective control icons.

In particular, said audio icons and said control icons are adjustable by an administrator user.

Advantageously, the administrator user acts on said audio icons and on said control icons also remotely by means of connection means.

### Description of the figures of the invention

Further characteristics and advantages of the invention will be better highlighted by examining the following detailed description of a preferred, but not exclusive, embodiment, illustrated by way of non-limiting example, with the support of the accompanying drawings, wherein:
figure 1 shows a block diagram identifying the main steps of the method for adjusting a track;
figure 2 shows schematically a screenshot in which there are represented icons representing the different audio tracks and the relative means for the adjustment of the individual audio tracks according to specific control parameters.

### Detailed description of the invention

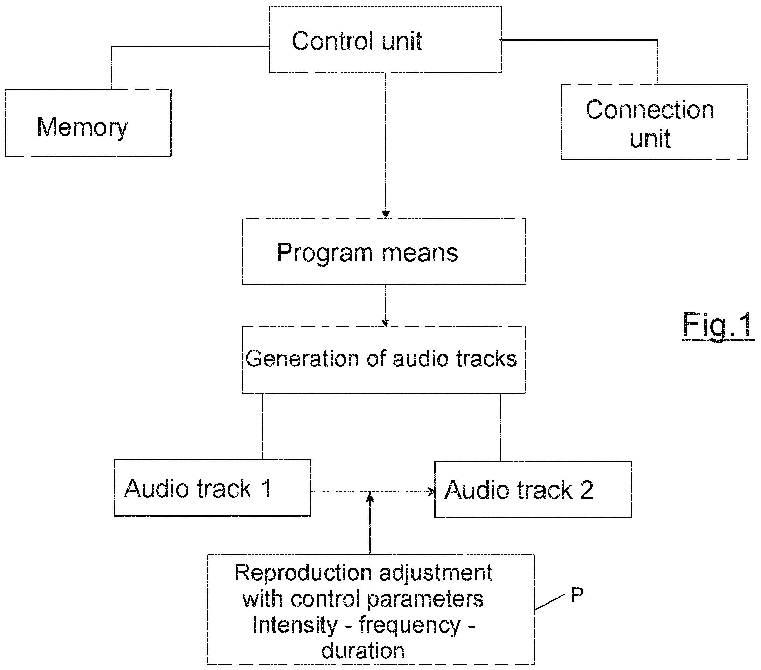

With reference to figure 1, there is schematically shown an audio track adjustment device that executes operational steps of a respective method according to the present invention.

The adjustment device comprises a control unit, a memory unit associated with the control unit. In the control unit there are provided programmed means which execute the adjustment method according to the present invention.

In particular, the adjustment method includes the steps of generating at least a first audio track and a second audio track. The first and second audio track are diversified from each other as a function of control parameters P selected from:
- Audio intensity of said first audio track with respect to said second audio track;
- Time duration of said first audio track with respect to a second audio track;
- Time interval or frequency between said first and second audio track;
- or a combination thereof.

In particular, the method according to the present invention allows to diversify according to the control parameters at least two audio tracks different from each other, generating a sound mismatch.

In particular, the control parameters are selected from:
- Intensity,
- Quality (tone: waveform),
- Frequency,
- Duration,
- Chronological distance
- Density,
- or a combination thereof.

In particular, the first and second audio track are reproduced in sequence with each other and diversified by varying the control parameters.

The generation of the mismatch can be based on a single control parameter or on several control parameters in combination with each other, by varying the difficulty of the sound exercise.

In other words, the adjustment method allows to obtain diversified audio tracks, i.e. single or multiple changes in the structure of the audio track itself or of the temporal type of one audio track with respect to another.

In fact, the perception of the mismatch by patients is the key element of the exercises, that is to sensitize the perception of difference between a first audio track with respect to a second audio track that was listened to immediately before, or in sequence. The method according to the present invention allows to generate audio tracks generating a mismatch based on variations of tone, volume and intensity in sound stimulations with the same quality. In fact, it has been observed that the mismatch sensation perceived is unchanged in people with brain injuries.

In the sound sequences, therefore, the control parameter creates the mismatch owing to what in the sequence itself was heard immediately before.

In particular, the method according to the present invention is basically based on the coexistence of at least three parallel audio tracks in constant relation, each subdivided into a maximum of nine regions groupable in a different way depending on the difficulty, for example according to a scheme of the type: 3+3+3, 4+4+3, 2+2+2+3, 1X9.

In particular, the time distance between each region is adjustable up to 10 seconds.

One can intervene on each region to create the mismatch.

Advantageously, the program means allow to generate a plurality of audio tracks distinct from each other, track 1, track 2 track n. In this way, the plurality of distinct audio tracks makes it possible to generate different sound exercises, each with a diversified degree of difficulty.

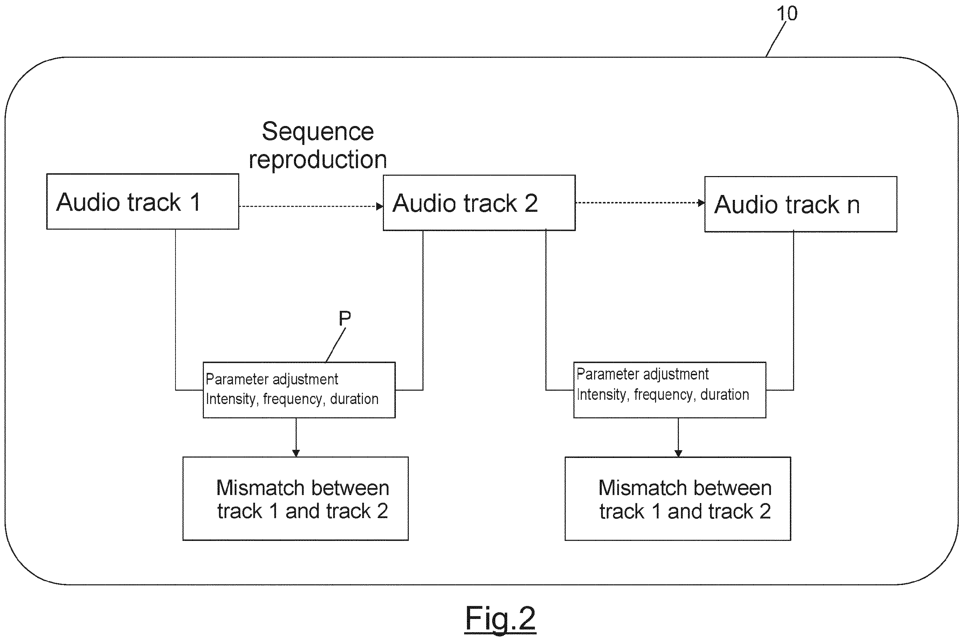

In particular, each of the control parameters P, as shown in figure 2, is adjustable by a user via a display screen 10 associated with the control unit.

In particular, different interfaces are provided to diversify the sound exercises. Preferably four different interfaces, with icons of different shape and/or colour are provided.

In particular, a step of choice among several themes is provided. The choice of the themes allows not to create repetitions, with the possibility of always adding new themes (within the limits of the memory of the devices) without having to intervene in terms of programming.

In addition, a choice of the difficulty level is allowed. In particular, among the following modes:
- Monody
- Level of basic difficulty, in which there is only a monodic melodic line without any harmonic `accompaniment'.
- Polyphony (1)
- Level of greater difficulty in which there is a melody with harmony: the mismatch occurs at both the harmonic and melodic levels
- Polyphony (2)
- Level of greater difficulty in which there is a melody with harmony: the mismatch occurs only at the melodic level.
- Polyphony (3)
- Level of greater difficulty in which there is a melody with harmony: the mismatch occurs only at the harmonic level.

Finally, there is the choice of the mismatch:
- Intensity: Volume variations
- Pitch; Frequency variations (octave difference so as not to create 'out of tune' but only frequency difference) in one or more regions of the sequence
- Tone: variation of tones of the instruments used in one or more regions of the sequence
- Harmony: sudden modulation in one or more regions of the sequence
- Rhythm: change in duration and/or distance between sounds (in a regular way) in one or more regions of the sequence
- Timing: change in duration and/or distance between sounds (in an irregular way) in one or more regions of the sequence.

In addition, these additional levels already fully ready at the level of themes and variation management are provided:
- Distance, duration, intensity (•)
- Distance duration (•)
- Intensity duration (•)
- Distance intensity (••)
- Duration (•)
- Intensity (••)
- distance (••)

In particular, in the exercises indicated with (•), sounds that have a long decadence are necessary (not the guitar for example); the choice was that of an electric bass that has a rhythmic function due to the percussiveness of the sound but also has a sufficient duration to manage the variation of the latter.

In the exercises indicated with (••), in which only these perceptual parameters are varied, the sound of a sharp "kick" is used, since the `duration' variable is not contemplated.

The control method according to the present invention therefore makes it possible to use the negative mismatch in a rehabilitation function, i.e. to exploit the structure of the sound language. The advantages of a control method that implements negative mismatch are:
- is universal and cancels language barriers. Everything can be used anywhere and at any age, without problems of language, culture, communication, indeed, an obstacle always present in cognitive rehabilitation that always requires communication.
- In cases of brain injuries, the ability to connect sounds in sequence remains intact.
- The link between the sounds in sequence is a mental operation of 'involuntary' type (like the beating of the heart at a physical level), so it does not create excessive effort (a sort of passive gymnastics), so much so that patients (tested and tried) are able to work for the entire duration of the therapeutic session (usually 30-40 minutes) unlike other standard cognitive exercises, so fatigue turns up after about ten to fifteen minutes).

To do this, tonal declination is used, for a historical matter of simplicity at the level of the functional structure (the themes, however, since they can be changed, can also be composed as a function of the preferred cultural environment, therefore with different formal and harmonic structures.

The above description of one or more specific embodiments is able to show the invention from a conceptual point of view so that others, using the known art, will be able to modify and/or adapt the embodiments in various applications without further research and without departing from the inventive concept, and, therefore, it is understood that such adaptations and modifications will be considered as equivalent to the specific embodiment. The means and materials for carrying out the various functions described may be of various kinds without thereby departing from the scope of the invention. It is understood that the expressions or terminology used are purely descriptive and therefore not limiting.

## Claims

1. Method for adjusting at least one audio track and which is executed by a control unit and a memory unit associated with the control unit, wherein said adjustment method comprises the steps of:
generating at least a first audio track and a second audio track, wherein said first and second audio track are diversified from each other as a function of control parameters selected from:
audio intensity of said first audio track with respect to said second audio track;
time duration of said first audio track with respect to a second audio track;
time interval between said first and second audio track;
or a combination thereof.

2. Method for adjusting control of an audio track according to claim 1, wherein program means allow to generate a plurality of audio tracks distinct from each other.

3. Method for adjusting an audio track according to claim 1 or 2, wherein each of said control parameters is adjustable by a user via a display screen associated with said control unit.

4. Method for adjusting an audio track according to one or more of the preceding claims, wherein the generation of at least three tracks reproduced in parallel with each other is provided, wherein each audio track is subdivided into a maximum of nine audio regions groupable in a different way depending on the difficulty, according to a scheme of the type: 3+3+3, 4+4+3, 2+2+2+3, 1X9, in particular, the time distance between each audio region is adjustable up to 10 seconds.

5. Audio track adjustment device comprising a control unit, a memory unit associated with the control unit, wherein in said control unit there are provided program means configured to:
generate at least a first audio track and a second audio track, wherein said first and second audio track are diversified from each other as a function of control parameters selected from:
audio intensity of said first with respect to said second audio track;
time duration of said first with respect to a second audio track;
time interval between said first and second audio track;
or a combination thereof.

6. Adjustment device according to claim 5, wherein a connection unit controlled by the control unit is provided, said connection unit allowing a remote control of said program means.

7. Adjustment device according to claims 5 or 6, wherein a display screen, in particular a touch screen, associated with said control unit is provided.
